# EUROPEAN PATENT SPECIFICATION

(11) **EP 0 684 986 B1**
(45) Date of publication and mention of the grant of the patent: **25.06.1997**
(21) Application number: 94906281.4
(22) Date of filing: 09.02.1994
(51) Int. Cl.: C12M 1/24, B01L 3/14

(54) **CAPS FOR CULTURE BOTTLES**
VERSCHLUSSKAPPEN FÜR KULTURFLASCHEN
COUVERCLES DESTINES A DES FLACONS DE CULTURE

(30) Priority: 17.02.1993 EP 93301173
(43) Date of publication of application: 06.12.1995
(73) Proprietor: UNIPATH LIMITED, Basingstoke, Hampshire RG24 OPW (GB)
(72) Inventor: CARR, Anthony Hugh, Stevington, Bedford MK43 7QS (GB)
(74) Representative: Matthews, Heather Clare
(86) International application number: GB9400256
(87) International publication number: WO9419452

(56) References cited:
- EP-A- 0 126 390
- EP-A- 0 391 461
- EP-A- 0 414 179
- US-A- 4 066 067

## Description

### Field of the invention

This invention relates to a cap for a culture bottle and more especially to an overcap which fits over the bottle seal.

### Background to the invention

Culture bottles used in microbiology typically have a seal crimped in place at the top of the bottle neck and an overcap (sometimes also referred as an overcover) fitted over the seal. The overcap is often coloured for identification purposes, and has a tear-off portion constituting a tamper-evident closure. A principal object of this invention is to provide an improved overcap for a culture bottle.

### Summary of the invention

According to one aspect of the present invention, there is provided an overcap for a culture bottle, said overcap comprising a principally cylindrical body having on its underside a round rim for snap-fitting over the culture bottle seal and at its top a round tear-off portion enabling the seal to be exposed, the body also having a lateral projection which has four generally right-angled corners.

The lateral projection of the cap, with its square corners, in particular facilitates safe handling of the culture bottle fitted with the overcap and also gives knowledge relevant to its orientation. This is important in relation to a preferred feature of the overcap, according to which the lateral projection has a substantially flat upper surface bearing a machine-readable identification. This machine-readable identification may conveniently be a barcode.

In a preferred embodiment, the lateral projection extends all round the body. The said lateral projection is preferably relatively thin and the body has cylindrical sections above and below the projection. Additionally, the projection, when the overcap is fitted to the bottle, preferably lies approximately in or just below the plane of the bottle seal.

In the preferred embodiment, the projection preferably has a port formed in it for a light indicator uniquely associated with the bottle cap in use. This port creates an asymmetry in the cap which dictates orientation, e.g. in relation to the machine-readable identification code, which is preferably located on the side of the projection opposite to the light-indicator port, which port is itself preferably located adjacent a corner of the lateral projection.

Again, especially in the preferred embodiment, the upper cylindrical section of the body, when the tear-off portion is removed, has a relatively deep throat which at the bottom engages the bottle seal. The bottom of the throat may conveniently be formed with a thin inwardly and downwardly directed deformable lip for engaging the seal. In this way, tolerances in manufacture are taken up. The throat is preferably formed near the bottom with an apertured inwardly projecting tab serving as a guide for the needle of a venting device. In this connection, it will be appreciated that a high pressure can in some circumstances build up in the culture bottle during use. This may be indicated by the light-indicating device viewable at the port previously referred to. The apertured tab ensures correct registration with the bottle of a venting device used for releasing the excess pressure.

According to another aspect of the invention, there is provided a culture bottle fitted with the overcap hitherto defined.

Additionally, the invention provides an incubation silo fitted with the culture bottle having the above-defined overcap. The lateral projection then provides the additional features of centring the bottle in the silo recess and closing the top of the recess to reduce temperature transients. Centring of the bottle in the correct orientation is important for several reasons, for example to facilitate reading of the machine-identifiable code on the lateral flange, and also to assist uniform heat transfer to the bottle within the silo.

The invention finds particular, but not exclusive, application in connection with an automated micro-organism growth monitoring system, eg as disclosed in PCT/GB92/01327 (WO93/03178).

A preferred embodiment of overcap in accordance with the invention will now be described, by way of example, with reference to the accompanying drawings, in which:-
Figures 1 and 2 show the overcap fitted to a culture bottle, in Figure 1 with a tear-off portion detached and in Figure 2 prior to tear off; and
Figure 3 shows the overcap in axial cross-section, after removal of the tear-off portion.

### Detailed Description of the Drawings

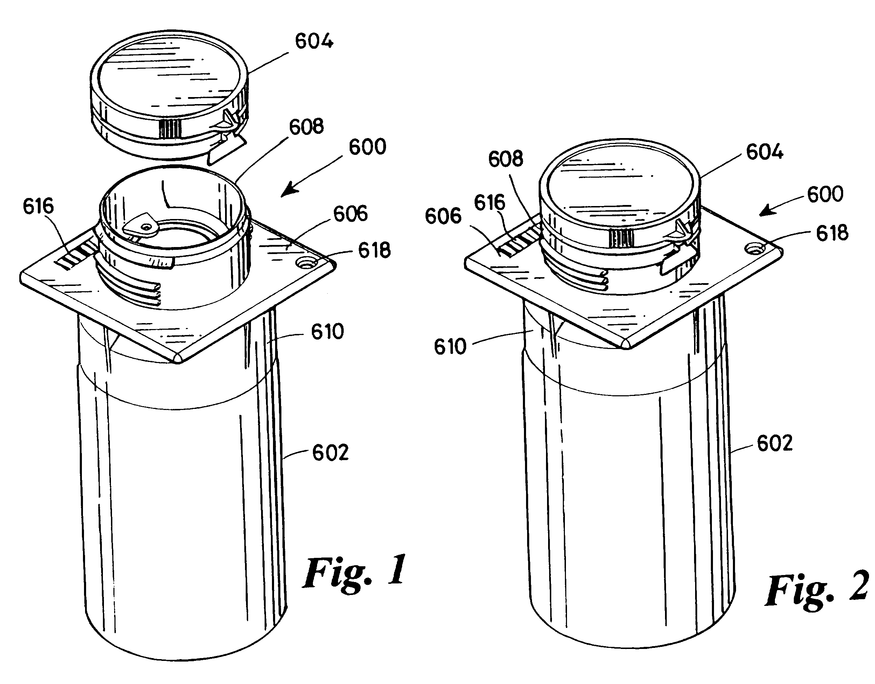

Referring to the drawings, an overcap 600 is shown fitted to a culture bottle 602, in Figure 1 with a tamper-evident tear-off portion 604 removed.

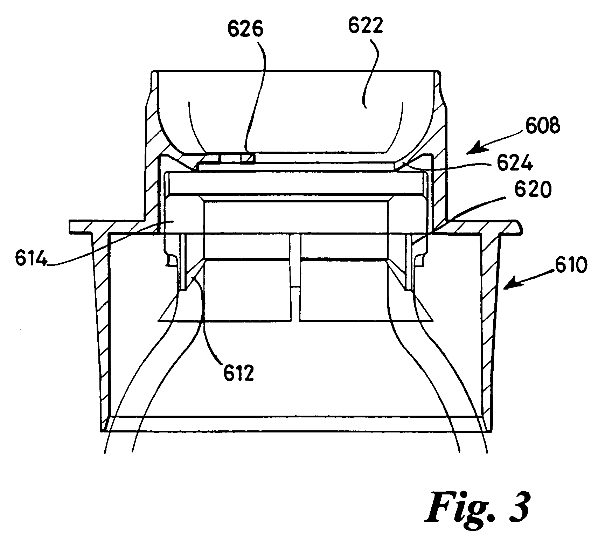

This preferred embodiment of overcap 600 has all round a generally square lateral projection 606 which is relatively thin, so that the body of the cap has cylindrical sections 608 and 610 respectively above and below the square projection. The lower cylindrical section 610 has internally a lip 612 which snap-fits over the bottle seal 614 (see Figure 3).

The upper surface of the projection 606 is on one side marked with a barcode 616 for bottle identification purposes, and on the opposite side, adjacent one corner, has a port 618 at which, when the bottle is in use in an incubation carrier, an LED uniquely associated with each particular bottle cap can be viewed. Thus, an LED may operate if excess pressure develops in the bottle with which the LED is uniquely associated and the bottle 602 may then be removed for special examination or treatment. Safe and easy handling, even by an operator wearing gloves, is facilitated by the square lateral projection 606, and the LED port 618 imparts a tactile and visible asymmetry which facilitates replacement of the bottle in the correct orientation, e.g. for reading of the barcode 616, which uniquely identifies the bottle independently of its position in the carrier. Additionally, the square lateral projection 606 ensures that the bottle is correctly centred in the silo recess in the carrier, thereby to assist uniform heat transfer to the bottle, and also covers the top of said silo recess to minimise temperature transients. In connection with both these advantages, it is to be appreciated that accurate temperature control of the liquid contents and gaseous headspace in the bottle is essential in microbiological testing. Ultrasensitive laser detection of septum displacements makes accurate temperature control more important than is usual.

Internally, as shown in Figure 3, the overcap 600 has an inner dependent sleeve 620 within the lower outer cylindrical section 610. The lip 612 which snap-fits over the bottle seal 614 is provided at the bottom of this inner sleeve 620.

Above the inner sleeve, the upper cylindrical section 608 is formed internally with a relatively deep throat 622, which at the bottom is terminated by a curved, inwardly and downwardly directed deformable lip 624 which engages the top of the bottle seal, taking up manufacturing tolerances. The deep throat 622, in conjunction with the square lateral projection 606, reduces risk of "needle-stick" injuries when a hypodermic syringe is used, for example to sample the bottle contents. Also adjacent the bottom of the throat 622, the cap 600 is formed internally with an apertured tab 626 which constitutes a guide for the needle of a bottle venting device. The guide tab ensures correct registration of the venting needle, ensuring that the scan path of the measurement laser is not obstructed.

The culture bottle 602 fitted with the overcap 600 is especially suitable for use in the monitoring system claimed and described for use in PCT/GB92/01327(WO 93/03178), wherein the bottle is one of a number of bottles located in silos in a multi-bottle carrier and the growth of microorganisms in the cultures is monitored by detecting displacement of the bottle septums (flexible diaphragms) responsively to pressure changes internally of the bottles. A preferred detection device is a laser which, by relative movement of the carrier and said laser, scans all the bottles in sequence repeatedly to measure displacements of the septums.

In such a system the formation of the overcap 600 with its square lateral projection 606, is important for facilitating handling of the bottles, for correctly orientating the bottles in the carrier, and for providing a flat upper sufrace which readily presents the barcode 616 to a machine reader, thus ensuring correct bottle identification even if during use it is for any reason removed from the carrier and replaced in a different position.

The overcap of Figures 1 to 3 is preferably manufactured in one piece by injection moulding.

## Claims

1. A plastics overcap (600) for a culture bottle (602), said overcap comprising a principally cylindrical body (608,610) having on its underside a round rim (612) for snap-fitting over the culture bottle seal (614) and at its top a round tear-off portion (604) enabling the seal to be exposed, the body also having a lateral projection (606) which has four generally right-angled corners.

2. An overcap as claimed in claim 1, wherein the lateral projection has a substantially flat upper surface bearing a machine-readable identification (616).

3. An overcap as claimed in claim 2, wherein the machine-readable identification is a barcode (616).

4. An overcap as claimed in claim 1, 2 or 3, wherein the lateral projection extends all round the body.

5. An overcap as claimed in claim 4, wherein the lateral projecton is relatively thin and the body has cylindrical sections (608,610) above and below the projection.

6. An overcap as claimed in claim 5, wherein the projection, when the overcap is fitted to the bottle, lies approximately in or just below the plane of the bottle seal.

7. An overcap as claimed in any one of claims 4 to 6, wherein the projection has a port (618) formed in it for a light indicator uiquely associated with the bottle cap in use.

8. An overcap as claimed in claim 5 or any claim appendant thereto, wherein the upper cylindrical section of the body (608), when the tear-off portion (604) is removed, has a relatively deep throat (622) which at the bottom engages the bottle seal.

9. An overcap as claimed in claim 8, wherein the bottom of the throat is formed with a thin inwardly and downwardly directed deformable lip (624) for engaging the seal.

10. An overcap as claimed in claim 8 or 9, wherein the throat is formed near the bottom with an apertured inwardly projecting tab (626) serving as a guide for the needle of a venting device.

11. A culture bottle (602) when fitted with the overcap of any of claims 1 to 10.

12. An incubation silo fitted with the culture bottle of claim 11, wherein the lateral projection of the overcap acts to centre the bottle within the silo recess.

13. An incubation silo fitted with the culture bottle of claim 11, wherein the lateral projection of the overcap serves to close the top of the silo recess.

## Patentansprüche

1. Kunststoff-Überzugverschlußkappe (600) für eine Kulturflasche (602), wobei die Überzugverschlußkappe einen im wesentlichen zylindrischen Körper (608, 610) aufweist, der an seiner Unterseite einen runden Rand (612) zum Einschnappen über den Verschluß (614) der Kulturflasche und an seiner Oberseite einen runden Abrißabschnitt (604) aufweist, der ermöglicht, daß der Verschluß freigelegt wird, wobei der Körper auch eine seitliche Auskragung (606) aufweist, die vier im wesentlichen rechtwinklige Ecken hat.

2. Überzugverschlußkappe nach Anspruch 1, wobei die seitliche Auskragung eine im wesentlichen flache obere Oberfläche aufweist, die eine maschinenlesbare Kennzeichnung (616) trägt.

3. Überzugverschlußkappe nach Anspruch 2, wobei die maschinenlesbare Kennzeichnung ein Strichcode (616) ist.

4. Überzugverschlußkappe nach Anspruch 1, 2 oder 3, wobei die seitliche Auskragung ganz um den Körper herum verläuft.

5. Überzugverschlußkappe nach Anspruch 4, wobei die seitliche Auskragung relativ dünn ist und der Körper zylindrische Abschnitte (608, 610) oberhalb und unterhalb der Auskragung aufweist.

6. Überzugverschlußkappe nach Anspruch 5, wobei die Auskragung, wenn die Überzugverschlußkappe auf der Flasche angebracht ist, etwa in oder unmittelbar unterhalb der Ebene des Flaschenverschlusses liegt.

7. Überzugverschlußkappe nach einem der Ansprüche 4 bis 6, wobei die Auskragung eine darin gebildete Öffnung (618) für eine der Flaschenverschlußkappe bei Benutzung eindeutig zugeordnete Lichtanzeige-Einrichtung aufweist.

8. Überzugverschlußkappe nach Anspruch 5 oder einem davon abhängigen Anspruch, wobei der obere zylindrische Abschnitt des Körpers (608), wenn der Abrißabschnitt (604) entfernt ist, einen relativ tiefen Hals (622) aufweist, der an der Unterseite mit dem Flaschenverschluß in Eingriff steht.

9. Überzugverschlußkappe nach Anspruch 8, wobei die Unterseite des Halses mit einer dünnen, nach innen und unten gerichteten deformierbaren Lippe (624) zum Eingriff mit dem Verschluß ausgebildet ist.

10. Überzugverschlußkappe nach Anspruch 8 oder 9, wobei der Hals nahe der Unterseite mit einer geöffneten, nach innen vorstehenden Durchführung (626), die als eine Führung für die Nadel einer Entlüftungsvorrichtung dient, ausgebildet ist.

11. Kulturflasche (602), die mit der Überzugverschlußkappe nach einem der Ansprüche 1 bis 10 ausgebildet ist.

12. Inkubationssilo, das mit der Kulturflasche nach Anspruch 11 ausgestattet ist, wobei die seitliche Auskragung der Überzugverschlußkappe zur Zentrierung der Flasche innerhalb der Ausnehmung des Silos dient.

13. Inkubationssilo, das mit der Kulturflasche nach Anspruch 11 ausgestattet ist, wobei die seitliche Auskragung der Überzugverschlußkappe zum Schließen des Oberteils der Ausnehmung des Silos dient.

## Revendications

1. Couvercle de recouvrement en matière plastique (600) pour flacon de culture (602), ledit couvercle de recouvrement comportant un corps principalement cylindrique (608, 610) ayant sur son côté inférieur une nervure circulaire (612) destinée à venir s'agencer par encliquetage sur le joint de flacon de culture (614) et au niveau de sa partie supérieure une partie de déchirement circulaire (604) permettant au joint d'être exposé, le corps ayant aussi une saillie latérale (606) qui a quatre coins de manière générale à angle droit.

2. Couvercle de recouvrement selon la revendication 1, dans lequel la saillie latérale a une surface supérieure à peu près plate supportant une identification pouvant être lue par une machine (616).

3. Couvercle de recouvrement selon la revendication 2, dans lequel l'identification pouvant être lue par une machine est un code à barres (616).

4. Couvercle de recouvrement selon la revendication 1, 2 ou 3, dans lequel la saillie latérale s'étend tout autour du corps.

5. Couvercle de recouvrement selon la revendication 4, dans lequel la saillie latérale est relativement mince et le corps a des tronçons cylindriques (608, 618) situés au-dessus et en dessous de la saillie.

6. Couvercle de recouvrement selon la revendication 5, dans lequel la saillie, lorsque le couvercle de recouvrement est agencé sur le flacon, se trouve approximativement dans le plan du joint du flacon ou juste en dessous de celui-ci.

7. Couvercle de recouvrement selon l'une quelconque des revendications 4 à 6, dans lequel la saillie a un orifice (615) formé dans celle-ci pour un indicateur lumineux associé de manière unique au couvercle de flacon en utilisation.

8. Couvercle de recouvrement selon la revendication 5 ou l'une quelconque des revendications dépendantes de celle-ci, dans lequel le tronçon cylindrique supérieur du corps (608), lorsque la partie de déchirement (604) est enlevée, a une gorge relativement profonde (622) qui au niveau de la partie inférieure vient en contact avec le joint du flacon.

9. Couvercle de recouvrement selon la revendication 8, dans lequel la partie inférieure de la gorge est munie d'une fine lèvre (624) pouvant être déformée dirigée vers l'intérieur et vers le bas pour venir en contact avec le joint.

10. Couvercle de recouvrement selon la revendication 8 ou 9, dans lequel la gorge est formée à proximité de la partie inférieure, une patte (626) faisant saillie vers l'intérieur, munie d'une ouverture, servant en tant que guide pour l'aiguille d'un dispositif de décompression.

11. Flacon de culture (602) lorsqu'il est muni du couvercle de recouvrement selon l'une quelconque des revendications 1 à 10.

12. Silo d'incubation muni du flacon de culture de la revendication 11, dans lequel la saillie latérale du couvercle de recouvrement agit pour centrer le flacon dans la cavité de silo.

13. Silo d'incubation muni du flacon de culture de la revendication 11, dans lequel la saillie latérale du couvercle de recouvrement sert à fermer la partie supérieure de la cavité de silo.
